Europäisches Patentamt

European Patent Office

Office européen des brevets

(19)

(11) Publication number: **0 129 419**
**B1**

(12)

# EUROPEAN PATENT SPECIFICATION

(45) Date of publication of patent specification: **09.09.87**

(51) Int. Cl.⁴: **G 01 N 33/38**, G 01 N 17/00

(21) Application number: **84304039.5**

(22) Date of filing: **15.06.84**

(54) A ceramic testing method.

(30) Priority: **17.06.83 JP 107645/83**

(43) Date of publication of application:
**27.12.84 Bulletin 84/52**

(45) Publication of the grant of the patent:
**09.09.87 Bulletin 87/37**

(84) Designated Contracting States:
**DE FR GB IT SE**

(56) References cited:
**DE-A-3 237 846**

(73) Proprietor: NGK INSULATORS, LTD.
2-56, Suda-cho, Mizuho-ku
Nagoya-shi, Aichi 467 (JP)

(72) Inventor: Soma, Takao
55-1 Aza-Ozakiyama Narumi-Cho Midori-Ku
Nagoya City (JP)
Inventor: Matsui, Minoru
193 Aza-Imonoshihora Ohaza-Kamiyashiro
Idaka-Cho
Meito-Ku Nagoya City (JP)

(74) Representative: Paget, Hugh Charles Edward
et al
MEWBURN ELLIS & CO. 2/3 Cursitor Street
London EC4A 1BQ (GB)

EP 0 129 419 B1

Courier Press, Leamington Spa, England.

## 0 129 419

**Description**

This invention relates to a ceramic testing method, and more particularly to a method of testing ceramics containing tetragonal zirconia.

Ceramics containing tetragonal zirconia have been extensively studied for years as materials for high-temperature structures, cutting tools, and oxygen sensors, because of their high heat resistivity, high mechanical strength and great toughness and their special properties of ionic conductivity at high temperatures.

While zirconia is stable in the tetragonal crystal phase at high temperatures, it is stable in the monoclinic crystal phase at low temperatures, so that when it is subjected to a temperature change in excess of a certain range, phase transformation is caused in zirconia accompanied with a considerable change in its volume.

As well known to those skilled in the art, in producing a ceramic containing zirconia, it is usually fired at a high temperature, so that the zirconia is in the tetragonal phase at the high temperature. In the process of being cooled, the tetragonal phase of zirconia is transformed into the monoclinic phase, and this transformation is accompanied by volumetric expansion which tends to form cracks in the material and lead to deterioration in the strength thereof.

To avoid this difficulty, it has been the practice to add oxides such as yttria and magnesia to zirconia or to control the microstructure of the sintered body, so as to maintain the tetragonal zirconia, which is stable at high temperatures, as a quasi-stable phase at low temperatures. In this way, the transformation from the tetragonal phase to the monoclinic phase of the crystal system is minimized, and the cracks due to such transformation are eliminated, so that strong ceramics containing zirconia can be produced. However, such quasi-stable tetragonal zirconia has the shortcoming that, although the zirconia is prevented from being transformed into its monoclinic phase for a short period of time, it is gradually transformed as time elapses, and deterioration such as the occurrence of cracks and reduction in mechanical strength may result. Efforts have been made to eliminate materials which are liable to such deterioration.

With conventional ceramic testing methods, it has been very difficult to foresee the possible deterioration of the material. In one conventional practice, the subject ceramics or materials are exposed to specific use conditions for a specific period of time, and then are tested to determine the occurrence of deterioration. This method, which is a kind of durability test, has the shortcoming that it is costly and requires a long test time. Thus with conventional ceramic testing methods, development of highly reliable ceramics containing zirconia has been difficult due to the lack of quickly obtainable test results, and users have to do without a guarantee of the reliability of the ceramic products.

An object of the present invention is to provide an improved ceramic testing method which enables quick determination of the reliability of ceramics containing tetragonal zirconia.

The ceramic testing method according to the present invention for a ceramic specimen having a zirconia content of not less than 5% by weight and containing tetragonal zirconia is characterized by heating the specimen in water or an atmosphere containing not less than 0.08 g/l water vapor for a predetermined period of time at a temperature in the range 50 to 1,250°C, prior to measuring a property or properties of the specimen.

Preferably, the water vapor atmosphere, if used, contains not less than 2.5 g/l of water. The ceramics preferably contains not less than 50% by weight of zirconia. The maximum limit of the zirconia content allowable in the ceramic testing method of the invention is 100% by weight. The preferred temperature range for the heating of the specimen is 150—500°C.

As described above, when a ceramic containing zirconia is fired at a high temperature, a fired body containing tetragonal zirconia is obtained. As the fired body is cooled, transformation from the tetragonal phase to the monoclinic phase occurs at a temperature below the thermodynamic equilibrium temperature between the tetragonal and monoclinic crystalline systems. This transformation tends to cause cracks and deterioration of properties such as mechanical strength and electrical characteristics. The probability of occurrence of the transformation of zirconia from its tetragonal phase to its monoclinic phase and the probability of deterioration of the ceramic containing tetragonal zirconia depend on various factors, and the actual degree of deterioration after use varies considerably depending on the materials and the manner of use.

The present inventors have found that a very important factor, which controls the deterioration of ceramics containing tetragonal zirconia due to the zirconia transformation from tetragonal phase to monoclinic phase, is the amount of water contained in the atmosphere. This is in addition to the conventionally known major factors affecting such deterioration i.e., the kinds and amounts of additives dissolved in solid phase in the zirconia, the magnitudes and amounts of tetragonal zirconia crystal grains, the temperatures maintained, and the periods for which such temperatures are maintained. The present invention is based on this finding.

The rate of deterioration of ceramics containing tetragonal zirconia increases with the increase of water content in the atmosphere. The details of the mechanism by which water accelerates this deterioration are not clear yet, but it appears that water reduces the surface energy of the monoclinic crystal so as to facilitate generation of crystal nuclei for the transformation.

The effect of water in accelerating deterioration is exhibited only in the case where a ceramic which

2

contains tetragonal zirconia becomes quasi-stable below the equilibrium temperature between the tetragonal and monoclinic phases. In the case of ceramics having no transformation, such as stabilized zirconia and alumina, the above-mentioned effect of water in accelerating deterioration is not exhibited. Thus, water relates to the transformation of tetragonal zirconia into monoclinic zirconia. Particularly, water has the effect of accelerating the deterioration of ceramics containing tetragonal zirconia.

To predict the nature of the deterioration of a ceramic containing tetragonal zirconia after actual use for a long period of time, one can put a sample of the ceramic in an atmosphere containing more water than that in the actual atmosphere in which it is used while maintaining it at a temperature suitable for causing the transformation, and then measure the degree of deterioration. In practice, for instance, a specimen and water are sealed in a closed vessel, and the vessel is heated from the outside and the vessel temperature is controlled at a suitable level, so as to keep the specimen in water or in water vapor atmosphere for a predetermined period of time.

The content of water in the atmosphere surrounding the specimen can be determined from the temperature held, the pressure held, the weight of water added, and the interior volume of the vessel, from consideration of the equilibrium of water and water vapor. In addition to pure water, if other substances such as alcohol and water glass are present, the content of water in the atmosphere during the test can be determined by separate measurements preceding the test, to obtain the water content in the atmosphere for different temperatures and pressures under similar conditions. It is also possible to determine the water content directly by applying Karl Fischer titration or another analytical process to a sample taken from the actual test atmosphere.

The vessel to which the specimen and water are to be added need not always be sealed. The specimen may be placed in an open vessel and then exposed to water or water vapor. In this case, the water content of the atmosphere can be determined by regular measurement of humidity of the atmosphere. The specimen can be for example a ceramic article as used or can be a piece of ceramics cut off from such article.

After exposure to the atmosphere containing water vapor, the specimen is preferably cooled, and then the degree of deterioration of the specimen can be determined by a suitable method; for example one or more of a visual inspection for checking the presence of cracks on the specimen surface, a dye-adsorption test for checking the presence of cracks by suitable dye such as red ink, crack detection using a supersonic defect detector or an x-ray penetration defect detector, measurement of the amount of transformation from tetragonal phase to monoclinic phase by measurement of thermal expansion hysteresis or x-ray diffraction measurement, measurement of strength, measurement of thermal shock resistance, measurement of shape, and an electrical test using measurement of ionic conductivity.

The reasons for numerical limitations of preferred conditions in the method of the invention will be given now. The water content in the atmosphere is not less than 0.08 g/l, because the water content of saturated steam at 50°C is about 0.08 g/l, so that if the water content in the atmosphere is less than 0.08 g/l, the atmosphere may have a low humidity comparable with that of the regular atmosphere in which the ceramic containing tetragonal zirconia is actually used, so that the desired effect of accelerating deterioration cannot be expected. If the water content in the atmosphere is in excess of 2.5 g/l, the resistivity against deterioration of the ceramics containing tetragonal zirconia can be evaluated in a short period of time. The preferable content of zirconia in the ceramics is not less than 5% by weight, because if the zirconia content is less no substantial improvement of the strength and toughness can be achieved. Not less than 50% by weight of zirconia is preferable, because in this case various properties inherent to zirconia can be revealed in the ceramics such as heat insulation, ionic conductivity, and resistivity against chemicals. The heating temperature is 50—1,250°C, because the deterioration usually occurs in this temperature range. The more preferred temperature range is 150—500°C, because the rate of deterioration is very high in this temperature range.

The single accompanying drawing is a schematic sectional view of a measuring system which is suitable for carrying out a ceramic testing method embodying the present invention.

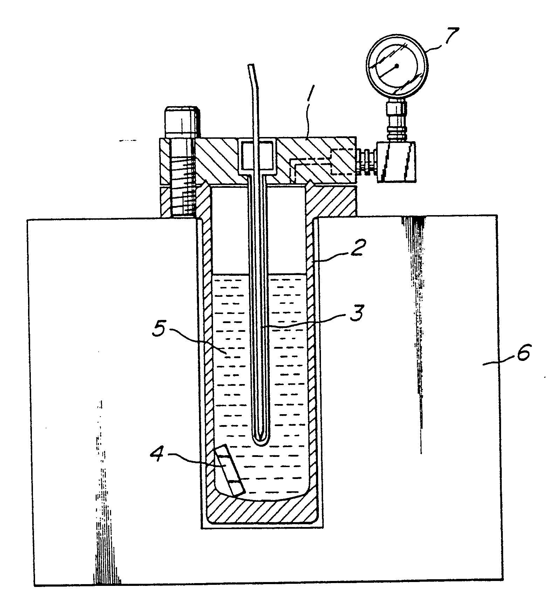

The drawing shows an autoclave 1 for testing a ceramic specimen by a method according to the present invention prior to testing its properties. The autoclave 1 has a vacuum casing 2 carrying a thermocouple 3, and a specimen 4 of the ceramic to be tested is placed in the vacuum vessel 2 together with water 5. A heater 6 surrounds the vacuum vessel 2, and a manometer 7 measures the pressure in the vacuum vessel 2.

The invention will now be illustrated in detail by several Examples.

Example 1

A starting powder material was prepared by weighing 91.8% by weight of commercially obtained zirconia and 8.2% by weight of commercially obtained yttria, mixing and pulverizing them in a ball mill for 50 hours, and drying the mixture thus pulverized. The starting powder material was shaped into plates, each plate having a width of 60 mm, a length of 60 mm, and a thickness of 6 mm, and a static hydraulic pressure of 1,000 kg/cm$^2$ was applied to them. Three groups of the thus shaped plates were fired at different temperatures for three hours; a first group at 1,400°C, a second group at 1,500°C, and a third group at 1,600°C.

Strength test specimens, each having a cross section of 3×4 mm and a length of 40 mm, were prepared

3

from the fired plates using a diamond cutter and a diamond grindstone in accordance with Japanese Industrial Standard (JIS) R 1601 "Test Method of Bending Strength of Fine Ceramics".

Disk specimens, each having a diameter of 20 mm and a thickness of 3 mm, for x-ray diffraction measurement were prepared, and their surfaces were finished by buffing so as to provide a surface roughness of less than 0.8 S as stipulated in JIS B 0601.

The strength test specimens were placed in the autoclave as shown in the accompanying drawing, so as to process them at certain temperatures for predetermined periods of time. For comparison, the specimens were placed in an electric furnace for effecting aging treatment in air at certain temperatures for predetermined periods of time. Both before and after these processes and treatments, various tests were carried out; i.e., visual inspections to check for cracks, dye-absorption tests to check for surface fine cracks, and strength tests. The strength tests were carried out by the four-point bending method as stipulated in JIS R 1601.

x-ray diffraction measurements were taken on the above-mentioned disk specimens using a copper (Cu) bulb x-ray diffraction device under the conditions of a bulb voltage of 50 kV, a bulb current of 80 mA, and a scanning speed of 0.25°/min. The presence of tetragonal zirconia was checked using the tetragonal zirconia ($ZrO_2$) peaks (200), (002), (004), and (220).

The result of the tests is shown in Table 1. In the table, under the heading of "dye-absorption test", the symbol A represents no exudation, the symbol B represents slight exudation, and the symbol C represents considerable exudation.

In the case of the zirconia porcelain used in the tests of this example, accelerated aging was noticed when specimens were heated at about 250°C in the presence of water, and the degree of the accelerated aging increased with the firing temperature. As shown in Table 1, when the zirconia porcelain specimens were heated at about 250°C in air having a normal humidity (a water content of 0.005—0.02 g/l), aging occurred only after heating over a long period in the order of 1,000 hours. If the same specimens were heated in an atmosphere with a high water content, similar aging could be caused in a very short period of time.

4

TABLE 1

| Test No. | Specimen | | | Aging test | | | | | Strength (MPa) | Cracks | Dye-absorption test*** |
| | Type of ceramics | Composition (% by Wt) | Tetragonal zirconia | Atmosphere | Water content in atmosphere (g/l) | Pressure Pa×10⁵ (Atm) | Temperature (°C) | Time (Hr) | | | |
|---|---|---|---|---|---|---|---|---|---|---|---|
| 1 | zirconia | | | in water** | 799 | 39.5 (39) | 250 | 10 | 820 | none | A |
| (2) | ceramics | | | none | | | | | 810 | none | A |
| (3) | A* | | | in air | 0.005—0.02 | 1 (1) | 250 | 1,000 | 800 | none | A |
| 4 | | | | in water** | 958 | 1 (1) | 100 | 10 | 840 | none | A |
| 5 | | | | ditto | 917 | 5.1 (5) | 150 | 10 | 720 | none | B |
| 6 | | | | ditto | 865 | 15.2 (15) | 200 | 10 | 340 | none | B |
| 7 | | | | ditto | 799 | 39.5 (39) | 250 | 1 | 530 | none | B |
| 8 | | | | ditto | 799 | 39.5 (39) | 250 | 10 | 210 | exist | C |
| 9 | | | | ditto | 799 | 39.5 (39) | 250 | 100 | 0 | exist | C |
| 10 | zirconia | $ZrO_2$:91.8 | contained | in steam** | 20.0 | 39.5 (39) | 250 | 10 | 470 | exist | C |
| 11 | ceramics | | | in water** | 712 | 86.1 (85) | 300 | 10 | 230 | exist | C |
| 12 | B* | $Y_2O_3$:8.2 | | ditto | 572 | 165 (163) | 350 | 10 | 600 | exist | B |
| (13) | | | | none | | | | | 830 | none | A |
| (14) | | | | in air | 0.005—0.02 | 1 (1) | 100 | 1,000 | 820 | none | A |
| (15) | | | | in air | 0.005—0.02 | 1 (1) | 250 | 100 | 830 | none | A |
| (16) | | | | in air | 0.005—0.02 | 1 (1) | 250 | 1,000 | 210 | exist | C |
| (17) | | | | in air | 0.005—0.02 | 1 (1) | 500 | 1,000 | 790 | none | A |
| (18) | | | | in air | 0.005—0.02 | 1 (1) | 800 | 1,000 | 850 | none | A |
| 19 | zirconia | | | in water** | 799 | 39.5 (39) | 250 | 10 | 0 | exist | C |
| (20) | ceramics | | | none | | | | | 780 | none | A |
| (21) | C* | | | in air | 0.005—0.02 | 1 (1) | 250 | 1,000 | 540 | exist | C |

Notes:
1. Test Nos. in brackets are examples outside the scope of the invention.
2. * Zirconia ceramics A, B, and C were fired at 1,400°C, 1500°C, and 1,600°C, respectively.
   ** In water in autoclave, and in steam in autoclave.
   *** In dye-absorption test, symbols A, B, and C represent no, slight, and much absorption, respectively.

Example 2

Specimens of various ceramics containing tetragonal zirconia were subjected to temperature cycle tests in air by raising and reducing the temperature at a rate of 200°C/hr in a range of 100°C to 1,000°C.

Similar specimens were heated at certain temperatures for a predetermined period of time in an atmosphere with a high water content, and then dye-absorption tests were performed on the specimens.

The specimens, conditions and results are shown in Table 2. For comparison, results of tests outside the scope of the invention are also shown in the table.

The test results show that the method of the invention was effective in quickly detecting those defective ceramics containing tetragonal zirconia which would be easily aged.

The test results on the comparison specimens of stabilized zirconia ceramics and alumina ceramics show that no aging was caused in those ceramics which did not contain tetragonal zirconia even after heating in an atmosphere with a high water content. Thus, the method of the present invention is clearly different from regular water corrosion tests, because the method of the invention is particularly useful in testing the aging of ceramics containing tetragonal zirconia.

TABLE 2

| Test No. | Specimen | | | Aging test | | | | | Dye-absorption test** |
|---|---|---|---|---|---|---|---|---|---|
| | Type of ceramics | Composition (% by Wt) | Tetragonal zirconia | Atmosphere | Water content in atmosphere (g/l) | Pressure Pa×$10^5$ (Atm) | Temperature (°C) | Time (Hr) | |
| 1 | zirconia | $Zr_2O_3$:93.2 | contained | in steam* | 20.0 | 39.5 (39) | 250 | 1 | C |
| 2 | ceramics | $Y_2O_3$:5.0 | contained | in water* | 799 | 39.5 (39) | 250 | 1 | C |
| (3) | D | $Al_2O_3$:1.8 | contained | in air | 0.005—0.02 | 1 (1) | note 3 | 100 | A |
| (4) | | | contained | in air | 0.005—0.02 | 1 (1) | note 3 | 1,000 | C |
| 5 | zirconia | $ZrO_2$:93.1 | contained | in water* | 799 | 39.5 (39) | 250 | 10 | A |
| (6) | ceramics | $Y_2O_3$:4.8 | contained | in air | 0.005—0.02 | 1 (1) | note 3 | 100 | A |
| (7) | E | $SiO_2$:2.1 | contained | in air | 0.005—0.02 | 1 (1) | note 3 | 1,000 | A |
| 8 | | | contained | in steam | 0.256 | 1 (1) | 600 | 100 | C |
| 9 | zirconia | $ZrO_2$:96.9 | contained | in water* | 799 | 39.5 (39) | 250 | 10 | C |
| (10) | ceramics | MgO:3.1 | contained | in air | 0.005—0.02 | 1 (1) | note 3 | 100 | A |
| (11) | F | | contained | in air | 0.005—0.02 | 1 (1) | note 3 | 1,000 | C |
| 12 | alumina | $Al_2O_3$:92.5 | contained | in water* | 799 | 39.5 (39) | 250 | 10 | C |
| (13) | zirconia | $ZrO_2$:7.5 | contained | in air | 0.005—0.02 | 1 (1) | note 3 | 100 | A |
| (14) | ceramics | | contained | in air | 0.005—0.02 | 1 (1) | note 3 | 1,000 | C |
| (15) | stabilized | | none | none | | | | | A |
| (16) | zirconia | $ZrO_2$:86.4 | none | in air | 0.005—0.02 | 1 (1) | note 3 | 1,000 | A |
| (17) | ceramics | $Y_2O_3$:13.6 | none | in water* | 799 | 39.5 (39) | 250 | 10 | A |
| (18) | | | none | none | | | | | A |
| (19) | alumina | $Al_2O_3$:100 | none | in air | 0.005—0.02 | 1 (1) | note 3 | 1,000 | A |
| (20) | ceramics | | none | in water* | 799 | 39.5 (39) | 250 | 10 | A |

Notes:
1. Test Nos. in brackets are examples outside the scope of the invention.
2. * In water in autoclave, and in steam autoclave.
   ** In dye-absorption test, symbols A, B, and C represent no, slight, and much absorption, respectively.
3. Temperature in aging test was cyclically changed between 100°C and 1,000°C.

**0 129 419**

As described in detail in the foregoing, the reliability of ceramics containing tetragonal zirconia can be determined in a very short period of time. Such ceramics containing tetragonal zirconia can have high resistivities against mechanical and thermal stresses; namely, normal thermal stress, thermal shock stress, repeated stress, and repeated thermal stress. Examples of the use of such ceramics containing tetragonal zirconia include engine cylinder liners, piston caps, cylinder heads, valves, valve guides, exhaust ports, rocker arms, auxiliary combustion chambers, tappets, and oxygen sensors. Besides, ceramics containing tetragonal zirconia are also useful as materials for acid-resistive pumps and other parts exposed to acids, alkalis, and various chemicals. Ceramics containing tetragonal zirconia are also useful as materials for cutting tools, such as surgical knives, scissors, normal knives, and the like.

The ceramic testing method according to the present invention facilitates research and development of ceramics containing tetragonal zirconia with a view to achieving high durability and high reliability. With the method of the invention, tests for quality control of production can be effected quickly, and the durability of the products may be guaranteed based on such tests.

## Claims

1. A ceramic testing method for a ceramic specimen having a zirconia content of not less than 5% by weight and containing tetragonal zirconia, characterized by heating the specimen in water or an atmosphere containing not less than 0.08 g/l water vapor for a predetermined period of time at a temperature in the range 50 to 1,250°C, prior to measuring a property or properties of the specimen.

2. A ceramic testing method as set forth in claim 1, wherein the specimen is heated in a water vapor atmosphere containing not less than 2.5 g/l of water.

3. A ceramic testing method as set forth in claim 1 or claim 2 wherein the ceramic specimen contains not less than 50% by weight of zirconia.

4. A ceramic testing method as set forth in any one of claims 1 to 3, wherein said heating is effected at a temperature in the range 150 to 500°C.

5. A ceramic testing method as set forth in any one of the preceding claims, wherein said ceramic specimen is resistant to mechanical and thermal stresses including normal thermal stress, thermal shock stress, repeated stress, and repeated thermal stress.

6. A ceramic testing method as set forth in any one of the preceding claims, wherein said ceramic specimen is resistant to acidic corrosion.

7. A ceramic testing method as set forth in any one of the preceding claims, wherein said ceramics specimen is resistant to alkaline corrosion.

## Patentansprüche

1. Verfahren zum Testen einer Keramikprobe, die einen Gehalt an Zirkon von nicht weniger als 5 Gewichtsprozent aufweist und tetragonales Zirkon enthält, dadurch gekennzeichnet, daß die Probe in Wasser oder in einer Atmosphäre, die nicht weniger als 0,08 g/l Wasserdampf enthält, für eine bestimmte Zeitdauer bei einer Temperatur von 50 bis 1250°C erhitzt wird, bevor eine oder mehrere Eigenschaften der Probe gemessen werden.

2. Verfahren nach Anspruch 1, wobei die Probe in einer wasserdampfhaltigen Atmosphäre erhitzt wird, die nicht weniger als 2,5 g/l Wasser enthält.

3. Verfahren nach Anspruch 1 oder 2, wobei die Keramikprobe nicht weniger als 50 Gewichtsprozent Zirkon enthält.

4. Verfahren nach einem der Ansprüche 1 bis 3, wobei das Erhitzen bei einer Temperatur von 150 bis 500°C durchgeführt wird.

5. Verfahren nach einem der vorhergehenden Ansprüche, wobei die keramische Probe gegen mechanische und thermische Belastungen, einschließlich normalen thermischen Belastungen, thermischen Stoßbelastungen, wiederholten Belastungen und wiederholten thermischen Belastungen beständig ist.

6. Verfahren nach einem der vorhergehenden Ansprüche, wobei die keramische Probe säurebeständig ist.

7. Verfahren nach einem der vorhergehenden Ansprüche, wobei die keramische Probe laugenbeständig ist.

## Revendications

1. Méthode pour tester la céramique pour un échantillon de céramique ayant une teneur en zircone de pas moins de 5% en poids et contenant de la zircone tétragonale, caractérisée en ce que l'on chauffe l'échantillon dans l'eau ou une atmosphère ne contenant pas moins de 0,08 g/l de vapeur d'eau pendant une période prédéterminée de temps à une température comprise entre 50 et 1250°C, avant de mesurer une ou plusieurs propriétés de l'échantillon.

2. Méthode pour tester la céramique selon la revendication 1, où l'échantillon est chauffé dans une atmosphère de vapeur d'eau ne contenant pas moins de 2,5 g/l d'eau.

3. Méthode pour tester la céramique selon la revendication 1 ou la revendication 2, où l'échantillon de céramique ne contient pas moins de 50% en poids de zircone.

4. Méthode pour tester la céramique selon l'une quelconque des revendications 1 à 3, où ledit chauffage est effectué à une température comprise entre 150 et 500°C.

5. Méthode pour tester la céramique selon l'une quelconque des revendications précédentes, où ledit échantillon de céramique est résistant aux efforts mécaniques et thermiques comprenant un effort thermique normal, un effort du choc thermique, un effort répété et un effort thermique répété.

6. Méthode pour tester la céramique selon l'une quelconque des revendications précédentes, où ledit échantillon de céramique est résistant à la corrosion acide.

7. Méthode pour tester la céramique selon l'une quelconque des revendications précédentes, où ledit échantillon de céramique est résistant à la corrosion alcaline.